(19)

Europäisches Patentamt
European Patent Office
Office européen des brevets

(11) **EP 4 272 730 A1**

(12) **EUROPEAN PATENT APPLICATION**
published in accordance with Art. 153(4) EPC

(43) Date of publication:
**08.11.2023 Bulletin 2023/45**

(21) Application number: **21916661.8**

(22) Date of filing: **11.06.2021**

(51) International Patent Classification (IPC):
*A61K 9/107* (2006.01)      *A61P 7/08* (2006.01)
*A61P 13/12* (2006.01)

(52) Cooperative Patent Classification (CPC):
**A61K 9/107; A61K 47/06; A61K 47/10;
A61K 47/14; A61K 47/22; A61K 47/24;
A61K 47/44; A61P 7/08; A61P 13/12**

(86) International application number:
**PCT/CN2021/099563**

(87) International publication number:
**WO 2022/147962 (14.07.2022 Gazette 2022/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.01.2021  CN 202110008666**

(71) Applicant: **SHANGHAI NINTH PEOPLE'S
HOSPITAL, SHANGHAI
JIAOTONG UNIVERSITY SCHOOL OF MEDICINE
Huangpu District
Shanghai 200011 (CN)**

(72) Inventors:
• **DING, Feng**
  **Shanghai 200011 (CN)**
• **WANG, Yifeng**
  **Shanghai 200011 (CN)**
• **SHEN, Yuqi**
  **Shanghai 200011 (CN)**
• **LI, Jiaolun**
  **Shanghai 200011 (CN)**

(74) Representative: **Canzler & Bergmeier
Patentanwälte
Partnerschaft mbB
Despag-Straße 6
85055 Ingolstadt (DE)**

(54) **FAT EMULSION DIALYSATE, AND PREPARATION METHOD THEREFOR AND USE THEREOF**

(57)    The present invention relates to the field of hemodialysis, in particular to a fat emulsion dialysate, and a preparation method therefor and the use thereof. Provided in the present invention is a fat emulsion dialysate, comprising long-chain fat emulsion oil, medium-chain triglyceride, an antioxidant, sodium oleate, glycerol, phospholipid and a solvent. Compared with traditional dialysis, the fat emulsion dialysate provided by the present invention clearly has a better removal advantage for protein-bound toxins. In addition, the fat emulsion dialysate not only has a simple preparation method and a low cost, but also has good stability and safety, and remains stable at room temperature for 14 days without obvious precipitation. Thus, the fat emulsion dialysate can become a new hemodialysis solution with broad application prospects, and has good industrialization prospects.

FIG. 1

EP 4 272 730 A1

**Description**

**FIELD OF DISCLOSURE**

The present invention relates to the field of hemodialysis, particularly, to a fat emulsion dialysate and a preparation method and use thereof.

**DESCRIPTION OF RELATED ARTS**

Protein-bound toxins refer to toxins that exist mostly in a bound form in plasma, and accumulate due to abnormal metabolism in a pathological state. Few protein-bound toxins are free in the blood, and protein-bound toxins cannot pass through the dialysis membrane, therefore, it is difficult to remove them by traditional hemodialysis technology. Accumulation of protein-bound uremic toxins (PBUTs) in patients with end-stage renal disease is associated with an increase in the incidence of uremic complications, where it has been proved that indoxyl sulfate and p-cresol sulfate are closely associated with an increase in all-cause mortality and cardiovascular disease incidence in patients with chronic kidney disease (CKD). Removal of uremic PBUTs is a difficult problem in blood purification technology. PBUTs are usually small molecular organic anions, in which organic anions with an aromatic phenyl ring mainly bind closely to site II of serum albumin in blood circulation, such as p-cresol sulfate (PCS), indoxyl sulfate (IS) and indolyl-3-acetic acid (3-IAA). For patients with abnormal liver function, bilirubin level is an independent risk factor affecting the death of patients with acute-on-chronic liver failure. The accumulated protein-bound toxins play an important role in the secondary development of serious complications such as renal failure, hepatic encephalopathy, and circulatory disorder. Artificial liver support technology (ALSS), represented by albumin dialysis (AD), came into being. ALSS can remove protein-bound toxic substances and metabolites such as bilirubin and bile acids accumulated in patients with liver failure to improve the internal environment, thus helping the patients to get through the acute phase until liver transplantation or self-liver function recovery.

During the treatment of albumin dialysis, free protein-bound toxins in blood diffuse into the dialysate so that the concentration of free toxins at the blood side decreases and the association/dissociation balance of toxin-albumin moves to the dissociation direction, thereby dissociating and releasing the toxins and achieving a new balance. The amount of toxins binding to albumin in the dialysate determines the amount of toxins to be removed. Albumin dialysis combines the advantage of protein-bound toxin removal by plasma infusion with good biocompatibility of hemodialysis, and is in nature a removal based on diffusion and adsorption. Even though albumin dialysis can effectively clear water-soluble toxins and protein-bound toxins and correct the internal environmental disorder in patients to mitigate the clinical symptoms, its clinical application is restricted by the resource of human albumin and the treatment cost. By looking for a blood purification technology which can substitute for albumin dialysis, has a low cost, and can effectively clear the protein-bound toxins, it will help to increase the removal of PBUTs in patients with uremia, and help to alleviate symptoms of patients with liver functional failure.

**SUMMARY OF THE PRESENT INVENTION**

In view of the shortcomings of the prior technology as set forth above, an object of the present invention is to provide a fat emulsion dialysate and a preparation method and use thereof for addressing the problems existing in the prior technology.

To achieve the above object and other objects, in an aspect, the present invention provides a fat emulsion dialysate, comprising a long-chain fat emulsion oil, a medium-chain triglyceride, an anti-oxidant, sodium oleate, glycerin, phospholipid, and a solvent.

In some embodiments of the present invention, the fat emulsion dialysate comprises, by weight percent,

3 to 15% of the long-chain fat emulsion oil;

1.5 to 9% of the medium-chain triglyceride;

0.05 to 0.3% of the anti-oxidant;

0.05 to 0.3% of sodium oleate;

0.2 to 2% of glycerin;

1 to 12% of phospholipid; and

65 to 95% of the solvent.

In some embodiments of the present invention, the long-chain fat emulsion oil is selected from one or more of olive oil and soybean oil;

and/or a total content of acid and decanoic acid in the medium-chain triglyceride is $\geq$ 99 wt%, preferably $\geq$ 99.5 wt%, and more preferably $\geq$ 99.9 wt%;

and/or the anti-oxidant is one or a combination of $\alpha$-tocopherol and squalene.

In some embodiments of the present invention, the phospholipid is selected from lecithins, preferably one or a combination of soybean lecithin and egg yolk lecithin.

In some embodiments of the present invention, the solvent is an aqueous solvent. The aqueous solvent comprises water, and further comprises one or more of sodium chloride, potassium chloride, calcium chloride, magnesium chloride, and glucose.

In some embodiments of the present invention, the fat emulsion dialysate is an oil-in-water system.

In some embodiments of the present invention, a fat emulsion particle size in the fat emulsion dialysate is from 150 to 500 nm.

In another aspect, the present invention provides a method for preparing the fat emulsion dialysate, comprising: providing an oil phase and an aqueous phase, mixing the oil phase with the aqueous phase, and homogenizing to provide the fat emulsion dialysate.

In some embodiments of the present invention, the mixing and/or homogenizing is carried out at a temperature of 50 to 90°C;

and/or the oil phase and the aqueous phase are thoroughly mixed by a high-speed shearing at a high-speed shear rate of 10000 to 20000 rpm and a shear time of 5 to 30 min;

and/or the homogenizing is carried at a pressure of 200 to 1000 bar.

In another aspect, the present invention provides a use of the fat emulsion dialysate for preparation of a hemodialysis dialysate.

## BRIEF DESCRIPTION OF THE DRAWINGS

FIG. 1 shows a schematic diagram of fat emulsion particles observed by a transmission electron microscope in Example 2 of the present invention.

FIG. 2 shows a schematic diagram showing a stability of the fat emulsion in Example 2 of the present invention.

FIG. 3 shows a schematic diagram of a capacity of fat emulsions with different phospholipid contents binding to three protein-bound toxins (p-cresol, IAA, and IS) in Example 3 of the present invention.

FIG. 4 shows a schematic diagram of a capacity of fat emulsion with 3% phospholipid content for removing three protein-bound toxins (p-cresol, IAA, and IS) in Example 4 of the present invention.

FIG. 5 shows a schematic diagram of an in vitro simulated dialysis device in Example 5 of the present invention.

FIG. 6 shows a schematic diagram of a capacity of fat emulsion with 3% phospholipid content for removing three protein-bound toxins (p-cresol, IAA, and IS) in Example 5 of the present invention under a condition of in vitro simulated dialysis.

FIG. 7 shows a schematic diagram of a cytotoxicity of fat emulsion in Example 7 of the present invention.

## DETAILED DESCRIPTION OF THE PREFERRED EMBODIMENTS

In order to make the purpose, technical solution, and beneficial technical effect of the present invention clearer, the present invention will be further described in detail by reference to the examples hereinafter. Persons skilled in the art can easily understand other advantages and efficacy of the present invention from the disclosure of the description.

Through a great number of practical research, the inventor provides a fat emulsion dialysate which can serve as a hemodialysis dialysate, and have a stronger binding and removal capacity to protein-bound toxins.

In a first aspect, the present invention provides a fat emulsion dialysate, comprising a long-chain fat emulsion oil, a medium-chain triglyceride, an anti-oxidant, sodium oleate, glycerin, phospholipid, and a solvent. The fat emulsion dialysate is commonly an emulsion system which utilizes a small-particle-sized oil phase as adsorptive material in dialysate to effectively enhance the binding capacity of the dialysate system to the protein-bound toxins (e.g., p-cresol sulfate (PCS), indoxyl sulfate (IS), indolyl-3-acetic acid (3-IAA), and the like), thereby effectively improving the protein-bound toxin removal capacity of the dialysate.

The fat emulsion dialysate provided by the present invention may comprise 3 to 15%, 3 to 4%, 4 to 5%, 5 to 6%, 6 to 7%, 7 to 8%, 7 to 9%, 9 to 10%, 10 to 11%, 11 to 12%, 12 to 13%, 13 to 14%, or 14 to 15% of the long-chain fat emulsion oil. The long-chain fat emulsion oil generally refers to a class of compounds comprising 17, 18, or 19 carbon atoms in the carbon chain of its primary ingredient fatty acid. In the fat emulsion dialysate, the long-chain fat emulsion oil mainly serves as one of the oil phase components. An appropriate amount of the long-chain fat emulsion oil can reduce the content of the oil phase to reduce the cost under the premise of ensuring the toxin removal effect. The long-chain fat emulsion oil is commonly pharmaceutical grade so that it can be suitable for the preparation of a dialysate. For example, it can meet the standards of the Chinese Pharmacopoeia 2015 edition, Second Volume. In particular, suitable long-chain fat emulsion oils may be one or more of olive oil, soybean oil, and the like.

The fat emulsion dialysate provided by the present invention may comprise, by weight percent, 1.5 to 9%, 1.5 to 2%, 2 to 2.5%, 2.5 to 3%, 3 to 3.5%, 3.5 to 4%, 4 to 4.5%, 4.5 to 5%, 5 to 5.5%, 5.5 to 6%, 6 to 6.5%, 6.5 to 7%, 7 to 7.5%, 7.5 to 8%, 8 to 8.5%, or 8.5 to 9% of the medium-chain triglyceride. The medium-chain triglyceride generally refers to a class of compounds with C8 to C10 fatty acids as primary ingredients (e.g., octanoic acid, decanoic acid, etc.), which can be extracted from coconut oil and/or palm oil. In the fat emulsion dialysate, the medium-chain triglyceride mainly serves as one of the oil phase components. In the fat emulsion dialysate, the presence of the medium-chain triglyceride generally helps to improve the oxidation stability of the oil phase. The medium-chain triglyceride is commonly pharmaceutical grade so that it can be suitable for the preparation of the dialysate. For example, it can meet the drug quality standards (YBH03422008). As another example, in the medium-chain triglyceride, the content of octanoic acid may be 55.4 to 61.4 wt%, 55.4 to 56.4 wt%, 56.4 to 57.4 wt%, 57.4 to 58.4 wt%, 58.4 to 59.4 wt%, 59.4 to 60.4 wt%, or 60.4 to 61.4 wt%; the content of decanoic acid may be 38.5 to 44.5 wt%, 38.5 to 39.5 wt%, 39.5 to 40.5 wt%, 40.5 to 41.5 wt%, 41.5 to 42.5 wt%, 42.5 to 43.5 wt%, or 43.5 to 44.5 wt%; and a total content of octanoic acid and decanoic acid is generally $\geq$ 99 wt%, $\geq$ 99.5 wt%, or $\geq$ 99.9 wt%.

The fat emulsion dialysate provided by the present invention may comprise, by weight percent, 0.05 to 0.3%, 0.05 to 0.1%, 0.1 to 0.15%, 0.15 to 0.2%, 0.2 to 0.25%, or 0.25 to 0.3% of the anti-oxidant. In the fat emulsion dialysate, the anti-oxidant mainly serves as one of the oil phase components. The anti-oxidant can effectively improve the colloidal stability of the fat emulsion. The anti-oxidant can be various anti-oxidants suitable for the preparation of the dialysate in the art. For example, the anti-oxidant may be one or more of $\alpha$-tocopherol, $\beta$-tocopherol, squalene (triacontahexaene).

The fat emulsion dialysate provided by the present invention may comprise, by weight percent, 0.05 to 0.3%, 0.05 to 0.1%, 0.1 to 0.15%, 0.15 to 0.2%, 0.2 to 0.25%, or 0.25 to 0.3% of sodium oleate. In the fat emulsion dialysate, the sodium oleate mainly serves as one of the aqueous phase components. The presence of sodium oleate serving as a supplement to the emulsifying capacity of phospholipid can improve the dispersibility and colloidal stability of the fat emulsion particles.

The fat emulsion dialysate provided by the present invention may comprise, by weight percent, 0.2 to 2%, 0.2 to 0.4%, 0.4 to 0.6%, 0.6 to 0.8%, 0.8 to 1%, 1 to 1.2%, 1.2 to 1.4%, 1.4 to 1.6%, 1.6 to 1.8%, or 1.8 to 2% of glycerin. In the fat emulsion dialysate, glycerin mainly serves as one of the aqueous phase components. The presence of glycerin can regulate the osmotic pressure of the fat emulsion dialysate.

The fat emulsion dialysate provided by the present invention may comprise, by weight percent, 1 to 12%, 1 to 2%, 2 to 3%, 3 to 4%, 4 to 5%, 5 to 6%, 6 to 7%, 7 to 8%, 8 to 9%, 9 to 10%, 10 to 11%, or 11 to 12% of phospholipid. In the fat emulsion dialysate, the phospholipid mainly serves as one of the aqueous phase components. In the fat emulsion dialysate, the increase of phospholipid content can generally result in an increase in the direct adsorption rate between the dialysate and the protein-bound toxins. The phospholipid may be various phospholipids suitable for the preparation of the dialysate, e.g., those meeting the standards of the U.S. Pharmacopoeia, the Japanese Pharmacopoeia, the European Pharmacopoeia, the Chinese Pharmacopoeia, and the like. In particular, suitable phospholipids may be lecithins (such as soybean lecithin and egg yolk lecithin) or the like.

The fat emulsion dialysate provided by the present invention may comprise, by weight percent, 65 to 95% of solvent, 65 to 70% of solvent, 70 to 75% of solvent, 75 to 80% of solvent, 80 to 85% of solvent, 85 to 90% of solvent, or 90 to 95% of solvent. In the fat emulsion dialysate, the aqueous components can generally dissolve in the solvent and constitute the aqueous phase of the emulsion system. In the fat emulsion dialysate, the solvent is generally an aqueous solvent. The aqueous solvent commonly comprises water, and water is commonly the main component of the aqueous solvent. In addition to water, the aqueous solvent can further comprise some components which can be introduced into the dialysate to meet the safety requirement of dialysis treatment. For example, the aqueous can further comprise one or more of sodium chloride (0.2 wt% to 0.8 wt%), potassium chloride (≤0.01 wt%), calcium chloride (0.01 wt% to 0.05 wt%), magnesium chloride (0.005 wt% to 0.03 wt%), glucose (0.15 wt% to 0.25 wt%) and the like.

In the fat emulsion dialysate provided by the present invention, the fat emulsion dialysate is commonly an oil-in-water system (O/W system), i.e., an emulsion system in which oil serving as an internal phase is dispersed in water serving as a continuous external phase. The oil phase is commonly uniformly distributed in the fat emulsion dialysate. The particle size of the oil phase (fat emulsion) may be appropriately 150 to 500 nm, 150 to 200 nm, 200 to 250 nm, 250 to 300 nm, 300 to 350 nm, 350 to 400 nm, 400 to 450 nm, or 450 to 500 nm.

In a second aspect, the present invention provides a method for preparing the fat emulsion dialysate provided by the first aspect of the present invention. On the basis that a formula of the fat emulsion dialysate is known, persons skilled in the art can properly select a method for preparing the fat emulsion dialysate. For example, the method may comprise providing an oil phase and an aqueous phase, mixing the oil phase with the aqueous phase, and homogenizing the mixture to provide the fat emulsion dialysate. As another example, when mixing and/or homogenizing, the temperature of the system may be 50 to 90°C, 50 to 60°C, 60 to 70°C, 70 to 80°C, or 80 to 90°C. As another example, the oil phase and the aqueous phase may be thoroughly mixed by high-speed shearing. The shear rate may be 10000 to 20000 rp/ min, 10000 to 15000 rp/ min, or 15000 to 20000 rp/ min, and the shear time may be 5 to 30 min, 10 to 20 min, 5 to 10 min, 10 to 12 min, 12 to 14 min, 14 to 16 min, 16 to 18 min, 18 to 20 min, 20 to 25 min, or 25 to 30min. As another example, the pressure condition of the homogenizing is 200 to 1000 bar, 200 to 400 bar, 400 to 600 bar, 600 to 800 bar, or 800 to 1000 bar. As another example, the pressure of the homogenizing may gradiently increase, and the homogenizing may be performed many times (e.g., 2 to 4 times) at the same pressure.

In a third aspect, the present invention provides a use of the fat emulsion dialysate provided by the first aspect of the present invention for preparation of a hemodialysis dialysate. As described above, the small-particle-sized oil phase of the system is utilized as adsorption material in the dialysate to effectively enhance the binding capacity of the dialysate system to the protein-bound toxins, so that the protein-bound toxin removal of the dialysate is improved, and the dialysate can be used for preparing the hemodialysis dialysate.

The fat emulsion dialysate provided by the present invention has a better advantage in protein-bound toxin removal as compared with conventional dialysis. Moreover, this fat emulsion dialysate is not only easy to prepare, has low cost, but also has good stability and safety. It maintains stable at room temperature for 14 days without obvious precipitation, and it can become a hemodialysis dialysate with wide application prospects and good industrialization prospects.

Hereinafter the invention of the present application is further described by reference to the examples, and it is not intended to limit the scope of the present application.

The information on the main reagents used in the examples is as follows:

Bovine serum albumin BSA (sigma, purity ≥ 98%);

P-cresol (sigma, CAS NO: 106-44-5);

Potassium indoxyl sulfate (IS, sigma, CAS NO: 2642-37-7);

Indolyl-3-acetic acid (3-IAA, sigma, CAS NO: 87-51-4);

Soybean oil (Aladdin, CAS NO: 8001-22-7);

Medium-chain triglyceride (MCT, C8: 58.4%, C10: 41.5%, C12: 0.1%);

Glycerin (CAS NO: 56-81-5);

Egg yolk lecithin (with a content of phosphatidylcholine of about 80%, CAS NO: 93685-90-6);

Sodium oleate (cis-9-octadecen-1-ol, CAS NO: 143-19-1);

$\alpha$-tocopherol (Aladdin, CAS NO: 59-02-9).

In the examples, high-performance liquid chromatography (HPLC) was used to establish the IS and PCS methodologies, and an Agilent Type 1100 HPLC was used to detect the concentrations of various toxins. During the detection, the samples comprising albumin and fat emulsion were processed by means of: sucking 200 µL of the sample solution, adding 600 µL of acetonitrile to precipitate the protein or fat emulsion, centrifuging at 4°C at 12,000 rpm for 20 min, and sampling the supernatant for detection.

The statistical method used in the examples was as follows: the detection results were processed with SPSS 21.0 statistical software, and the data was expressed by mean ± standard deviation. Independent-sample t test was used for comparison between the two groups, and a single-factor analysis of variance was used for comparison among multiple groups, $p < 0.05$ was considered statistically significant.

Example 1

An aqueous phase and an oil phase were prepared, respectively. 5.5 g of soybean oil, 4.5 g of medium-chain triglyceride, and 0.15 g of α-tocopherol were weighed, mixed, and stirred so that they were fully dissolved to serve as the oil phase. 0.13 g of sodium oleate, 0.9 g of glycerin, and a certain mass percent (0.4, 0.8, 1, 2, and 3%) of egg yolk lecithin were weighed, added into a certain volume of solution (water or dialysate), and stirred so that they were uniformly dissolved to serve as the aqueous phase. The oil phase was pretreated by high-speed shearing. The temperature of the aqueous phase was raised to 70°C, and then the oil phase was slowly injected into the aqueous phase. The mixture was subjected to high-speed shearing for 10 min. The obtained solution after shearing was subjected to gradient homogenization (600, 800, and 1000, twice at each gradient) under a pressure of 600 to 1000 bar by using a high-pressure homogenizer to provide a fat emulsion dialysate. The fat emulsion dialysate was used in cooperation with a 5% sodium bicarbonate solution during in vitro simulated dialysis. The specific ratio was 6.25 mL of 5% sodium bicarbonate solution per 100 mL of the fat emulsion dialysate.

Example 2

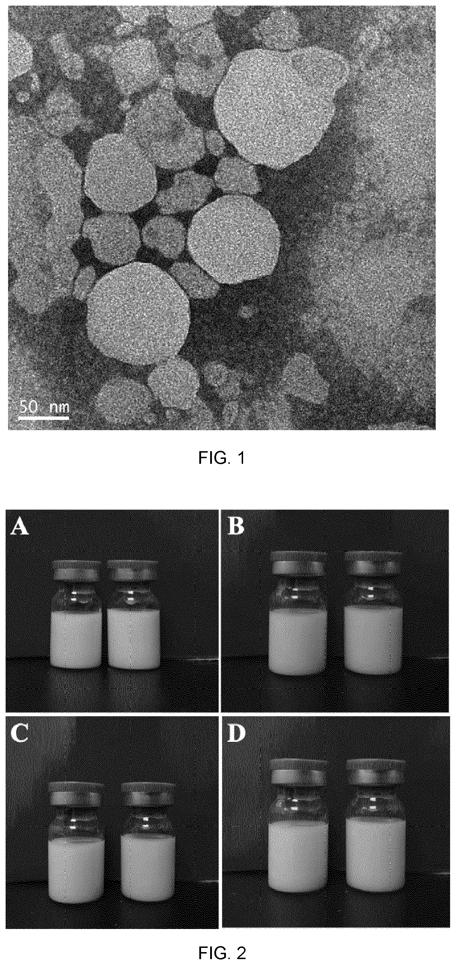

The hydration kinetic size of the fat emulsion in the fat emulsion dialysates with different phospholipid contents prepared according to the method described in Example 1 was detected by Malvern particle size analyzer, and the results were shown in Table 1. The surface morphology of the fat emulsion particles with 3% phospholipid content was observed by a transmission electron microscope, and the results were shown in FIG. 1. The apparent morphology (stability) of the fat emulsion during standing at room temperature for 21 days was shown in FIG. 2 (where A: Day 1; B: Day 4; C: Day 7; D: Day 14).

Table 1. The Particle Size of Fat Emulsions with Different Phospholipid Contents

| Phospholipid Content, % | Particle Size of Fat Emulsion, nm |
| --- | --- |
| 0.4 | 199.9 |
| 0.8 | 181.2 |
| 2.0 | 170.5 |

Example 3

The binding rate to PBUTs was detected as follows:
The fat emulsion dialysates with different phospholipid contents (1%, 2%, and 3%) prepared in Example 1 were selected, and the working concentrations of the protein-bound toxins were set as p-cresol (200 μmol/L), IS(150 μmol/L),and IAA(20 μmol/L),respectively. According to the above concentrations, the toxins were added into the fat emulsion dialysates with different phospholipid concentrations, and co-cultured for 1 hour. 400 μL of the co-culture solution were added into an ultrafiltration tube (Millipore Company, Mw = 3 K), and subjected to centrifugation at 25°C at 12000 rpm for 30 minutes. The toxin concentrations in the samples before and after ultrafiltration and the toxin concentrations of the filtrate were respectively detected by HPLC (n = 5), and the direct adsorption rates of the materials to the toxins were calculated. The calculation formula was as follows:

Direct Adsorption Rate% = (Total Concentration Before Ultrafiltration - Concentration of Filtrate After Ultrafiltration)/Total Concentration Before Ultrafiltration × 100%

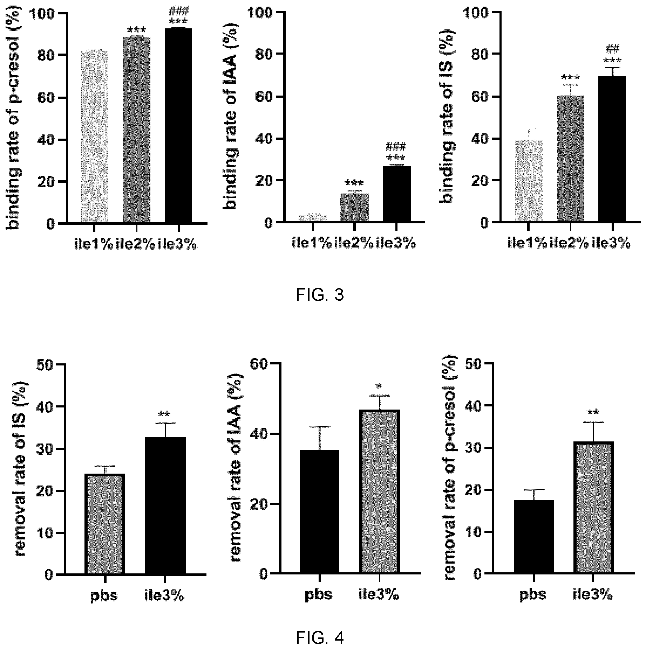

The detection results of the binding rate to PBUTs were specially shown in FIG. 3, where ile1%, ile2%, and ile3% were the 50 g/L fat emulsions with phospholipid content of 1%, 2%, and 3%, respectively. ***P < 0.001 as compared with the ile1%; and ##p<0.01, ###p < 0.001 as compared with the ile2%. As seen in FIG. 3, 50 g/L fat emulsions with phospholipid contents of 1%, 2%, and 3% have binding rates to the IS of about 39.4%, 60.42%, and 69.9%, respectively, have binding rates to the IAA of about 3.83%, 13.82%, and 27.06%, respectively, and have binding rates to the p-cresol of about 82.4%, 88.76%, and 92.61%, respectively. It can be seen that with the increase of phospholipid content, the direct adsorption rate of the fat emulsion to the protein-bound toxin increases.

Example 4

The removal effect to protein-bound toxins was evaluated by Equilibrium Plate Dialysis:
According to the results of direct adsorption rate, the fat emulsion dialysate with a phospholipid concentration of 3% (the same as Example 3) was selected for the next test. The indirect removal capacity of the material to various protein-bound toxins was evaluated by Rapid Equilibrium Dialysis (RED) (Thermo company, Mw=8 K). 40 g/L of HSA solution was prepared, and added to the protein-bound toxins p-cresol (200 $\mu$mol/L),IS(150 $\mu$mol/L), and IAA(20 $\mu$mol/L),respectively, followed by incubation at room temperature for 1 hour. Subsequently, 300 $\mu$l of HSA solution containing various protein-bound toxins as described above was added into the sample chamber of the RED plate, and 500 $\mu$l of fat emulsion with 3% phospholipid concentration or common sodium bicarbonate replacement solution (negative control) was added to the dialysate chamber. The RED was placed on a shaker, dialyzed at equilibrium at 37°C and 250 rpm for 4 hours. The samples were taken for detection (n = 5). Samples were taken from the sample chamber and dialysate chamber, respectively, and the toxin concentration was detected by HPLC. The indirect removal rate to toxin was calculated by the following formula:

$$\text{Toxin removal rate (\%)} = (\text{Toxin concentration at the sample side}$$
$$\text{before the dialysis - Toxin concentration at the sample side after}$$
$$\text{dialysis})/\text{Toxin concentration at the sample side before the dialysis} \times 100\%$$

The results of removal effect to protein-bound toxins evaluated by equilibrium plate dialysis were shown in FIG. 4. As seen in FIG. 4, the indirect removal rates of common sodium bicarbonate replacement solution to IS, IAA, and p-cresol were about 24.28%, 35.15%, and 17.62% respectively, while the indirect removal rates of fat emulsion with 3% phospholipid concentration to IS, IAA, and p-cresol were about 32.8% ($p<0.01$), 47.02% ($p<0.05$), and 31.52% ($p<0.01$), respectively. It can be seen that 50 g/L of fat emulsion can significantly improve the removal rate to protein-bound toxins as compared with common sodium bicarbonate replacement solution.

Example 5

The removal effects to protein-bound toxins were evaluated by simulated hemodialysis in vitro:

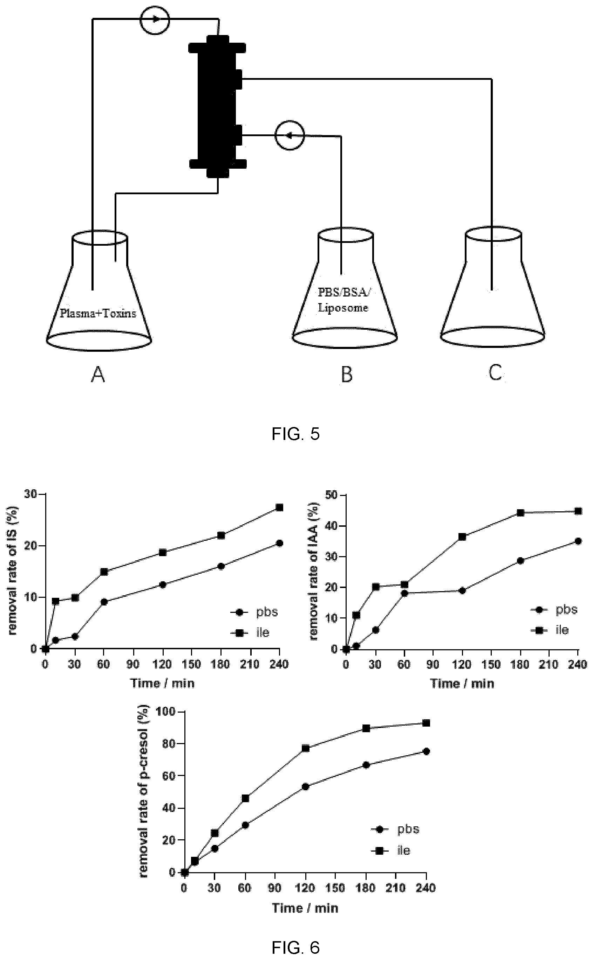

An in vitro dialysis model containing a small dialyzer with polysulfone membrane (Mw=20 KDa) (as shown in FIG. 5) was constructed to simulate the hemodialysis in vitro. 40g/L of BSA solution was prepared, and added to the protein-bound toxins p-cresol (200 $\mu$mol/L), IS (150 $\mu$mol/L), and IAA (20 $\mu$mol/L), respectively, followed by incubation at room temperature for 1 hour. Then 40 mL of BSA solution containing the protein-bound toxins was added to the blood side, and the fat emulsion dialysate with phospholipid concentration of 3% or common sodium bicarbonate dialysate was added to the dialysate side. The flow rate was set to 5 ml/min at the blood side, and 2.5 ml/min at the dialysate side. Samples were taken from the blood side and the dialysate side at 0, 10, 30, 60, 120, 180, and 240 minutes after extracorporeal circulation, and the concentrations of various toxins in the samples were measured. The change rules of various toxin concentrations at the blood and dialysate sides during extracorporeal hemodialysis within 4 hours were evaluated. The dialysis removal rates of common sodium bicarbonate dialysate and the fat emulsion dialysate to the various toxins were calculated and compared. The results were particularly shown in FIG. 6.

It can be seen from FIG. 6 that the removal rates to IS, IAA, and p-cresol of the common dialysate group were about 20.53%, 35.13%, and 75.34%, respectively, while the removal rates to IS, IAA, and p-cresol of the fat emulsion dialysate group were about 27.45%, 44.83%, and 92.99%, respectively by in vitro simulated hemodialysis for 4 hours. It can be seen that the removal effect of the fat emulsion dialysate group is higher than that of the common dialysate group.

Example 6

Hemolytic Test:

The biocompatibility of the fat emulsion was verified by studying the passive hemolysis behavior of the fat emulsion. Red blood cells collected from fresh human blood were diluted to 5%(v/v) by using a PBS solution. 300 $\mu$L of red blood cell suspension and 500 $\mu$L of the fat emulsion with 3% phospholipid concentration, PBS (negative control) or ddH2O (positive control) solution were added to the blood side and the sample side of the RED plate, respectively, and then shaken at 37°C for 4 hours. After the completion of the experiment, the red blood cells were collected, and centrifuged at 3700 rpm for 5 min. 100 $\mu$L of supernatant was added to a 96-well plate. At the detection wavelength of 541 nm, the absorbance (OD value) of the fat emulsion group, the positive control group, and the negative control group was detected, which were designated as $A_{test}$, $A_{control}$, and $A_{blank}$, respectively. The passive hemolysis of the fat emulsion was calculated according to the following formula.

$$\text{Hemolysis (\%)} = (A_{test} - A_{blank})/(A_{control} - A_{blank}) \times 100\%$$

According to the experimental results, the passive hemolysis of the fat emulsion was 0.60±1.33%.

Example 7

Cytotoxicity Test:

The biocompatibility of the fat emulsion with a phospholipid concentration of 3% was studied by CCK-8 cytotoxicity test using mouse embryonic fibroblasts (NIH-3T3). Cells were inoculated into a 96-well plate with a density of 10000, and grew adherently for 24 h. The culture medium was replaced by an ordinary DMEM medium or a DMEM medium solution containing the fat emulsion with a phospholipid concentration of 3% (0.1%, v/v). After 24 hours of co-culture, 10 $\mu$L of CCK-8 solution was added to the corresponding wells, and the cells were cultured at 37 °C for an additional 1 hour. The absorbance values (OD values, recorded as ATest) were detected at 450 nm. The OD values of blank wells and cell wells with the pure medium were detected (recorded as ABlank and ACTR, respectively). Cytotoxicity was calculated according to the following formula:

$$\text{Cytotoxicity (\%)} = (A_{Test} - A_{Blank}) / (A_{CTR} - A_{Blank}) \times 100\%$$

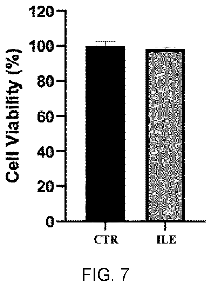

The experimental results were shown in FIG. 7. According to the experimental results, after co-incubation with the fat emulsion, the cell viability was 98.4±0.86%, showing good biocompatibility.

To sum up, the present invention effectively overcomes various shortcomings in the prior art and has high industrial utilization value.

The above examples are only for exemplarily illustrating the principles and efficacy of the present invention, but not for restricting the present invention. Any person skilled in the art can make modifications or variations to the above examples without departing the spirit and scope of the present invention. Thus, all the equivalent modifications or variations made by persons of ordinary skills in the related technical field without departing the spirit and technical concept disclosed by the present invention are still within the appended claims of the present invention.

**Claims**

1. A fat emulsion dialysate, comprising a long-chain fat emulsion oil, a medium-chain triglyceride, an anti-oxidant, sodium oleate, glycerin, phospholipid, and a solvent.

2. The fat emulsion dialysate of claim 1, wherein the fat emulsion dialysate comprises, by weight percent,

   3 to 15% of the long-chain fat emulsion oil;
   1.5 to 9% of the medium-chain triglyceride;
   0.05 to 0.3% of the anti-oxidant;
   0.05 to 0.3% of sodium oleate;

0.2 to 2% of glycerin;
1 to 12% of phospholipid; and
65 to 95% of the solvent.

3. The fat emulsion dialysate of claim 1, wherein the long-chain fat emulsion oil is selected from one or a combination of olive oil and soybean oil; and/or

a total content of acid and decanoic acid in the medium-chain triglyceride is no less than 99 wt%, preferably no less than 99.5 wt%, and more preferably no less than 99.9 wt%; and/or
the anti-oxidant is one or a combination of $\alpha$-tocopherol and squalene.

4. The fat emulsion dialysate of claim 1, wherein the phospholipid is selected from lecithins, preferably one or a combination of soybean lecithin and egg yolk lecithin.

5. The fat emulsion dialysate of claim 1, wherein the solvent is an aqueous solvent, the aqueous solvent comprises water, and further comprises one or more of sodium chloride, potassium chloride, calcium chloride, magnesium chloride, and glucose.

6. The fat emulsion dialysate of claim 1, wherein the fat emulsion dialysate is an oil-in-water system.

7. The fat emulsion dialysate of claim 6, wherein a fat emulsion particle size in the fat emulsion dialysate is 150 to 500 nm.

8. A method for preparing the fat emulsion dialysate of any one of claims 1 to 7, comprising: providing an oil phase and an aqueous phase, mixing the oil phase with the aqueous phase, and homogenizing to provide the fat emulsion dialysate.

9. The method for preparing the fat emulsion dialysate of claim 8, wherein the mixing and/or homogenizing is carried out at a temperature of 50 to 90°C; and/or

the oil phase and the aqueous phase are thoroughly mixed by high-speed shearing at a high-speed shear rate of 10000 to 20000 rpm and a shear time of 5 to 30 min; and/or
the homogenizing is carried at a pressure of 200 to 1000 bar.

10. Use of the fat emulsion dialysate of any one of claims 1 to 7 for preparation of a hemodialysis dialysate.

FIG. 1

FIG. 2

FIG. 3

FIG. 4

FIG. 5

FIG. 6

FIG. 7

## INTERNATIONAL SEARCH REPORT

International application No.

**PCT/CN2021/099563**

### A. CLASSIFICATION OF SUBJECT MATTER

A61K 9/107(2006.01)i;  A61P 7/08(2006.01)i;  A61P 13/12(2006.01)i

According to International Patent Classification (IPC) or to both national classification and IPC

### B. FIELDS SEARCHED

Minimum documentation searched (classification system followed by classification symbols)

A61K9/-;  A61P7/-;  A61P13/-

Documentation searched other than minimum documentation to the extent that such documents are included in the fields searched

Electronic data base consulted during the international search (name of data base and, where practicable, search terms used)

CNABS, CNTXT, VEN, DWPI, USTXT, EPTXT, WOTXT, CNKI, web of science, 读秀, 万方, 百度, 百度学术; 上海交通大学医学院, 丁峰, 王宜峰, 沈毓琪, 脂肪乳, 透析, 长链, 橄榄油, 大豆油, 中链甘油三酯, MCT, 生育酚, 角鲨烯, 油酸钠, 甘油, 磷脂, 华瑞制药, Shanghai Jiaotong University, lipid or fat?, emulsion?, dialy+ , soybean oil?, lecithin?, choline?, glycerin?, oleate, tocopher+, squalene

### C. DOCUMENTS CONSIDERED TO BE RELEVANT

| Category* | Citation of document, with indication, where appropriate, of the relevant passages | Relevant to claim No. |
|---|---|---|
| X | CN 1965806 A (SINO-SWED PHARMACEUTICAL CO., LTD.) 23 May 2007 (2007-05-23) description page 2 line 2 to page 7 line 20 | 1-9 |
| Y | CN 1965806 A (SINO-SWED PHARMACEUTICAL CO., LTD.) 23 May 2007 (2007-05-23) description page 2 line 2 to page 7 line 20 | 10 |
| Y | SHI Y.Y. et al. "Improved dialytic removal of protein-bound uremic toxins by intravenous lipid emulsion in chronic kidney disease rats" *Nephrol Dial Transplant*, Vol. 34, No. 11, 09 May 2019 (2019-05-09), ISSN: 0931-0509, abstract, discussion | 10 |
| X | CN 102348469 A (STABLE SOLUTIONS L.L.C.) 08 February 2012 (2012-02-08) description, table 1 | 1-9 |
| A | CN 101244037 A (GUANGZHOU FUBANG PHARMACEUTICAL TECHNOLOGY CO., LTD.) 20 August 2008 (2008-08-20) entire document | 1-10 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 July 2021** | **16 August 2021** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/099563**

**C. DOCUMENTS CONSIDERED TO BE RELEVANT**

| Category* | Citation of document, with indication, where appropriate, of the relevant passages | Relevant to claim No. |
|---|---|---|
| A | CN 1376460 A (SINO-SWED PHARMACEUTICAL CO., LTD.) 30 October 2002 (2002-10-30)<br>entire document | 1-10 |
| A | CN 111821263 A (SHANGHAI NINTH PEOPLE'S HOSPITAL) 27 October 2020 (2020-10-27)<br>entire document | 1-10 |
| A | CN 101312712 A (BIONTECH AG et al.) 26 November 2008 (2008-11-26)<br>entire document | 1-10 |
| A | CN 101190248 A (FAN, Min) 04 June 2008 (2008-06-04)<br>entire document | 1-10 |
| A | CN 109589306 A (SHANGHAI NINTH PEOPLE'S HOSPITAL) 09 April 2019 (2019-04-09)<br>entire document | 1-10 |
| A | WO 2020018512 A1 (NANOGEN LAB INC.) 23 January 2020 (2020-01-23)<br>entire document | 1-10 |
| A | 施雪枫等 (SHI, Xuefeng et al.). "脂质体血液透析清除蛋白结合性毒素的初步试验 (Preliminary Examination on Removal of Protein-Bound Toxins by Hemolipodialysis)" 复旦学报(医学版) (Fudan University Journal of Medical Sciences), Vol. 33, No. 2, 31 March 2006 (2006-03-31), ISSN: 1672-8467,<br>entire document | 1-10 |

Form PCT/ISA/210 (second sheet) (January 2015)

**INTERNATIONAL SEARCH REPORT**
Information on patent family members

International application No.

**PCT/CN2021/099563**

| Patent document cited in search report | | | Publication date (day/month/year) | Patent family member(s) | | | Publication date (day/month/year) |
|---|---|---|---|---|---|---|---|
| CN | 1965806 | A | 23 May 2007 | None | | | |
| CN | 102348469 | A | 08 February 2012 | JP | 2012520296 | A | 06 September 2012 |
| | | | | US | 8241672 | B2 | 14 August 2012 |
| | | | | CA | 2755084 | C | 03 February 2015 |
| | | | | CN | 102348469 | B | 17 December 2014 |
| | | | | PL | 2320949 | T3 | 30 April 2014 |
| | | | | CA | 2755084 | A1 | 16 September 2010 |
| | | | | MX | 2011009507 | A | 21 February 2012 |
| | | | | KR | 101503970 | B1 | 18 March 2015 |
| | | | | PL | 2320949 | T5 | 30 April 2018 |
| | | | | BR | PI1008935 | A2 | 15 March 2016 |
| | | | | EP | 2320949 | B2 | 13 December 2017 |
| | | | | KR | 20110130465 | A | 05 December 2011 |
| | | | | RU | 2011141102 | A | 20 April 2013 |
| | | | | EP | 2320949 | B1 | 23 October 2013 |
| | | | | PT | 2320949 | E | 07 November 2013 |
| | | | | WO | 2010104575 | A3 | 24 February 2011 |
| | | | | JP | 5860704 | B2 | 16 February 2016 |
| | | | | ES | 2443150 | T3 | 18 February 2014 |
| | | | | WO | 2010104575 | A2 | 16 September 2010 |
| | | | | NO | 2320949 | T3 | 22 March 2014 |
| | | | | IL | 214844 | A | 29 February 2016 |
| | | | | EP | 2320949 | A4 | 29 February 2012 |
| | | | | ES | 2443150 | T5 | 13 March 2018 |
| | | | | RU | 2555771 | C2 | 10 July 2015 |
| | | | | IL | 214844 | D0 | 30 November 2011 |
| | | | | US | 2010233280 | A1 | 16 September 2010 |
| | | | | EP | 2320949 | A2 | 18 May 2011 |
| | | | | IN | 315839B | B | 17 December 2014 |
| | | | | IN | 201106819 | P1 | 07 December 2012 |
| CN | 101244037 | A | 20 August 2008 | None | | | |
| CN | 1376460 | A | 30 October 2002 | None | | | |
| CN | 111821263 | A | 27 October 2020 | None | | | |
| CN | 101312712 | A | 26 November 2008 | WO | 2007070307 | A3 | 24 January 2008 |
| | | | | JP | 2009518424 | A | 07 May 2009 |
| | | | | US | 2007154498 | A1 | 05 July 2007 |
| | | | | IN | 200801422 | P2 | 03 April 2009 |
| | | | | AU | 2006324155 | A1 | 01 May 2008 |
| | | | | BR | 200619598 | A2 | 11 October 2011 |
| | | | | KR | 20080073288 | A | 08 August 2008 |
| | | | | CA | 2625640 | A1 | 21 June 2007 |
| | | | | WO | 2007070307 | A2 | 21 June 2007 |
| | | | | MX | 2008006946 | A1 | 30 June 2008 |
| | | | | SG | 143492 | A1 | 29 July 2008 |
| | | | | EP | 1957039 | A2 | 20 August 2008 |
| CN | 101190248 | A | 04 June 2008 | CN | 101190248 | B | 12 June 2013 |
| CN | 109589306 | A | 09 April 2019 | WO | 2019062071 | A1 | 04 April 2019 |
| WO | 2020018512 | A1 | 23 January 2020 | CA | 3106840 | A1 | 23 January 2020 |
| | | | | US | 2021015786 | A1 | 21 January 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 106-44-5
- *CHEMICAL ABSTRACTS,* 2642-37-7
- *CHEMICAL ABSTRACTS,* 87-51-4
- *CHEMICAL ABSTRACTS,* 8001-22-7
- *CHEMICAL ABSTRACTS,* 56-81-5
- *CHEMICAL ABSTRACTS,* 93685-90-6
- *CHEMICAL ABSTRACTS,* 143-19-1
- *CHEMICAL ABSTRACTS,* 59-02-9